Europäisches Patentamt

European Patent Office

Office européen des brevets

(19)

(11) **EP 1 316 303 A1**

(12) # DEMANDE DE BREVET EUROPEEN

(43) Date de publication:
**04.06.2003 Bulletin 2003/23**

(51) Int Cl.⁷: **A61K 7/48**

(21) Numéro de dépôt: **02292809.7**

(22) Date de dépôt: **12.11.2002**

(84) Etats contractants désignés:
**AT BE BG CH CY CZ DE DK EE ES FI FR GB GR IE IT LI LU MC NL PT SE SK TR**
Etats d'extension désignés:
**AL LT LV MK RO SI**

(30) Priorité: **28.11.2001 FR 0115375**

(71) Demandeur: **L'OREAL**
**75008 Paris (FR)**

(72) Inventeurs:
• **Biatry, Bruno**
  **94300 Vincennes (FR)**
• **Lheureux, Eric**
  **91230 Montgeron (FR)**

(74) Mandataire: **Rasson, Catherine**
**L'OREAL-DPI**
**6 rue Bertrand Sincholle**
**92585 Clichy Cedex (FR)**

(54) **Utilisation cosmétique et/ou dermatologique d'une composition contenant au moins un actif hydrophile sensible à l'oxydation stabilisé par au moins un polymère ou copolymère de N-vinylimidazole**

(57) L'invention se rapporte à l'utilisation cosmétique et/ou dermatologique d'une composition contenant, dans un milieu physiologiquement acceptable comprenant une phase aqueuse, au moins un actif hydrophile sensible à l'oxydation choisi parmi l'acide ascorbique et ses dérivés et au moins un polymère ou copolymère de N-vinylimidazole non réticulé, ledit actif et ledit polymère ou copolymère étant tous deux dans la phase aqueuse, pour prévenir ou lutter contre les effets néfastes des UV et/ou de la pollution sur la peau, en particulier la perte de fermeté et/ou d'élasticité de la peau.

EP 1 316 303 A1

**Description**

La présente invention se rapporte à l'utilisation cosmétique et/ou dermatologique d'une composition contenant au moins un actif hydrophile sensible à l'oxydation et au moins un polymère ou copolymère de N-vinylimidazole , dans un milieu physiologiquement acceptable comprenant une phase aqueuse.

Il est connu d'introduire dans des compositions cosmétiques divers actifs destinés à apporter des traitements spécifiques à la peau et/ou aux cheveux. Toutefois, certains de ces actifs présentent l'inconvénient d'être instables en milieu aqueux et de se dégrader facilement au contact de l'eau, en particulier à cause de phénomènes d'oxydation. Ils perdent ainsi rapidement leur activité au cours du temps et cette instabilité va à l'encontre de l'efficacité recherchée.

On cherche ainsi depuis longtemps à formuler l'acide ascorbique ou vitamine C, du fait de ses nombreuses propriétés bénéfiques. En particulier, l'acide ascorbique stimule la synthèse du tissu conjonctif et notamment du collagène, renforce les défenses du tissu cutané contre les agressions extérieures telles que les rayonnements ultraviolets et la pollution, compense la déficience en vitamine E de la peau, dépigmente la peau et possède une fonction anti-radicaux libres. Ces deux dernières propriétés en font un excellent candidat comme actif cosmétique ou dermatologique pour lutter contre le vieillissement de la peau ou prévenir celui-ci. Malheureusement, en raison de sa structure chimique (d'alpha-cétolactone), l'acide ascorbique est très sensible à certains paramètres de l'environnement et notamment aux phénomènes d'oxydation. Il s'ensuit donc une dégradation rapide de l'acide ascorbique formulé en présence de ces paramètres, et plus particulièrement en présence d'oxygène, de lumière, d'ions métalliques, en fonction de la température, ou encore dans certaines conditions de pH (Pharm. Acta. Helv., 1969, 44, 611-667 ; STP Pharma, 1985, 4, 281-286).

Plusieurs solutions ont donc été envisagées dans l'art antérieur pour diminuer et/ou retarder la dégradation de l'acide ascorbique.

Il a ainsi été proposé d'utiliser l'acide ascorbique sous forme de dérivé chimique (ascorbyl phosphate de magnésium, ou esters d'acides gras et d'acide ascorbique), mais la biodisponibilité de ces dérivés est très faible (J. Am. Acad. Dermatol., 1996, 34, 29-33).

L'instabilité de l'acide ascorbique vis à vis de l'oxygène a pu être améliorée en utilisant des conditionnements particuliers comme des bi-compartiments sous atmosphère inerte tels que décrits dans le brevet US-5,935,584, ou bien encore par l'utilisation d'émulsions à deux phases dont l'une est constituée d'une poudre sèche contenant l'acide ascorbique et la seconde d'une phase liquide. Le mélange des deux phases doit s'effectuer au moment de l'utilisation (WO98/43598). Ces solutions présentent des inconvénients au niveau du coût et de la complexité des fabrications ainsi que des contraintes importantes au niveau de l'utilisation.

Une autre solution proposée dans l'art antérieur consiste à utiliser des glycols ou des polyols en forte concentration afin de diminuer la solubilité de l'oxygène dans la formulation, protégeant ainsi l'acide ascorbique (WO-96/24325, EP 0 755 674, US-5,981,578). Les polyols peuvent éventuellement être incorporés dans des liposomes comme décrit dans le brevet US-6,020,367. Mais ces solutions présentent l'inconvénient de conduire à des formulations collantes dont la cosméticité est difficile à améliorer. Par ailleurs la présence d'une forte concentration de ces composés peut provoquer des phénomènes d'irritation.

L'acide ascorbique peut également être formulé dans des milieux anhydres tels que les silicones (US-6,194,452) qui sont capables de créer une barrière anhydre autour de l'acide ascorbique. Un inconvénient majeur de telles solutions résulte du manque de fraîcheur à l'application.

Il subsiste donc le besoin d'une composition utilisable notamment dans le domaine cosmétique, dans laquelle un actif hydrophile et instable en milieu oxydant est stabilisé, qui soit confortable lors de l'application, qui ne provoque aucune irritation de la peau après application, et qui soit compatible avec les contraintes d'une mise en oeuvre industrielle de son procédé de fabrication.

Le vieillissement cutané, de nature physiologique, peut-être accéléré par des facteurs environnementaux tels qu'une exposition répétée de la peau à la lumière solaire, et notamment aux rayonnements ultraviolets A, à la pollution, en particulier ou particules de diesel ou à la fumée de cigarette. L'action de l'environnement sur les constituants de la peau (fibres, cellules, enzymes) et sur le sébum sécrété par la peau entraîne en particulier la formation de radicaux libres oxygénés. Or, ces radicaux provoquent des dégâts oxydatifs importants, notamment dans les membranes cellulaires (perméabilité des membranes), les noyaux des cellules (destruction de l'ADN), et les tissus, en particulier le tissu conjonctif (dégradation des fibres d'élastine et de collagène). Ces dégâts conduisent notamment à une perte de fermeté et d'élasticité de la peau.

L'acide ascorbique, en piégeant les radicaux libres, protège les molécules biologiques. Ainsi appliqué par voie topique, il peut s'accumuler dans la peau et rétablir la concentration en acide ascorbique perturbée, par exemple dans le cas d'une exposition aux rayonnements UV. Il peut, d'autre part, protéger la peau des dommages induits par les UV (British Journal of Dermatology, 127, 1992, p. 247-253) ou par les stress environnementaux.

Parallèlement à cette activité, l'acide ascorbique présente un effet inhibiteur sur la sécrétion des cytokines pro-inflammatoires IL1$\alpha$ et IL6 induite par les UV (Journal of Investigative Derlatology, 108(3), 1997, p. 302-308). Il présente donc un intérêt particulier en tant q'agent apaisant des érythèmes solaires.

Le but de la présente invention est de proposer une composition contenant un actif sensible à l'oxydation choisi parmi l'acide ascorbique et ses dérivés, présentant de bonnes propriétés cosmétiques, tant au niveau du toucher qu'au niveau de la tolérance, dont la conservation dans le temps ne demande pas de précautions particulières, et qui conserve l'activité anti-radicalaire dudit actif, pour prévenir ou lutter contre les effets néfastes des UV et/ou de la pollution sur la peau, en particulier la perte de fermeté et/ou d'élasticité de la peau.

La Demanderesse a découvert, de manière fortuite, que l'utilisation de polymères ou copolymères de N-vinylimidazole non réticulés dans des compositions dont la phase aqueuse renferme un actif sensible à l'oxydation, tel que l'acide ascorbique ou un de ses dérivés, permettait d'atteindre le but précité.

Dans l'art antérieur, certains composés possédant une structure imidazole ont été décrits pour leurs propriétés stabilisatrices. Ainsi, dans la demande de brevet EP 0 586 106, plusieurs molécules à base d'imidazole sont utilisées pour stabiliser certains rétinoïdes contre les dégradations chimiques. D'autre part, des émulsifiants polymériques constitués de N-vinylimidazole, d'acrylates d'alkyles et de vinyl-acétates sont décrits dans le brevet US-4,057,622. Ils sont utilisés dans le but de remplacer les émulsifiants connus afin de pallier leurs inconvénients, en particulier au niveau de l'odeur, et pour stabiliser les émulsions eau-dans-huile. Enfin des copolymères de N-vinylimidazole / N-vinylcaprolactam / N-vinylpyrrolidone sont décrits dans le brevet US-6,191,188. Ils entrent dans la fabrication de compositions renforçantes pour les cheveux.

A la connaissance de la demanderesse, des polymères ou copolymères comportant des unités N-vinylimidazoles n'ont jamais été associés à des actifs hydrophiles sensibles aux dégradations par oxydation dans le but d'améliorer leur stabilité en milieu aqueux. Ceci est vrai en particulier dans le cas de l'acide ascorbique.

La présente invention a donc pour objet l'utilisation cosmétique et/ou dermatologique pour prévenir ou lutter contre les effets néfastes des UV et/ou de la pollution sur la peau, d'une composition, contenant, dans un milieu physiologiquement acceptable comprenant une phase aqueuse, au moins un actif hydrophile sensible à l'oxydation choisi parmi l'acide ascorbique et ses dérivés, et au moins un polymère ou copolymère de N-vinylimidazole non réticulé, ledit actif et ledit polymère ou copolymère étant tous deux dans la phase aqueuse. Le copolymère est présent en quantité suffisante pour stabiliser ledit actif hydrophile sensible à l'oxydation.

La présente invention a également pour objet l'utilisation cosmétique et/ou dermatologique pour prévenir ou lutter contre la perte de fermeté et/ou d'élasticité de la peau, d'une composition, contenant, dans un milieu physiologiquement acceptable comprenant une phase aqueuse, au moins un actif hydrophile sensible à l'oxydation choisi parmi l'acide ascorbique et ses dérivés, et au moins un polymère ou copolymère de N-vinylimidazole non réticulé, ledit actif et ledit polymère ou copolymère étant tous deux dans la phase aqueuse.

La présente invention a aussi pour objet l'utilisation d'une association, constituée, d'au moins un actif hydrophile sensible à l'oxydation choisi parmi l'acide ascorbique et ses dérivés, et d'au moins un polymère ou copolymère de N-vinylimidazole non réticulé, dans la phase aqueuse d'une composition cosmétique en tant qu'agent anti-radicalaire.

La présente invention a aussi pour objet l'utilisation d'une association, constituée, d'au moins un actif hydrophile sensible à l'oxydation choisi parmi l'acide ascorbique et ses dérivés, et d'au moins un polymère ou copolymère de N-vinylimidazole non réticulé, dans la phase aqueuse d'une composition cosmétique en tant qu'agent apaisant des érythèmes solaires.

Par "pollution", on entend aussi bien la pollution "extérieure", due par exemple aux particules de diesel, à l'ozone ou aux métaux lourds, que la pollution "intérieure" qui peut notamment être due aux émissions de solvants de peinture, de colles de moquette, d'isolants ou de papiers peints (tels que toluène, styrène, xylène ou benzaldéhyde), ou bien à la fumée de cigarette. Tous ces polluants sont en effet susceptibles de générer, directement ou indirectement, des radicaux libres.

Selon l'invention, par actif hydrophile on entend un composé ayant une solubilité dans l'eau d'au moins 0,25 % à température ambiante (25°C).

Selon l'invention, par actif hydrophile *sensible à l'oxydation* on entend tout actif d'origine naturelle ou synthétique susceptible de subir une dégradation par un mécanisme d'oxydation. Ce phénomène d'oxydation peut avoir plusieurs causes, en particulier la présence d'oxygène, de lumière, d'ions métalliques, une température élevée, ou encore certaines conditions de pH.

On peut citer à titre d'exemple et de façon non limitative : l'acide ascorbique et ses dérivés tels que les sels ou les esters, et particulièrement le 5,6-di-O-diméthylsilylascorbate (vendu par la Sté Exsymol sous la référence PRO-AA), le sel de potassuim du dl-alpha-tocopheryl-dl-ascorbyl-phosphate (vendu par la Sté Senju Pharmaceutical sous la référence SEPIVITAL EPC), l'ascorbyl phosphate de magnésium, l'ascorbyl phosphate de sodium (vendu par la Sté Roche sous la référence Stay-C 50).

Dans un aspect particulièrement avantageux, l'actif hydrophile sensible à l'oxydation est l'acide ascorbique.

Selon l'invention, par polymère ou copolymère de N-vinylimidazole non réticulé on entend tout polymère comportant des unités N-vinylimidazole, et ne comportant pas d'agent réticulant. Des copolymères convenant à la mise en oeuvre de l'invention sont les copolymères associant des sous unités N-vinylimidazole avec des sous unités N-vinylpyrrolidone et/ou N-vinylcaprolactam.

Dans un aspect avantageux de l'invention, le copolymère possède une fraction molaire en unité N-vinylimidazole comprise entre 0,1 et 1, et plus préférentiellement entre 0,4 et 0,9.

Selon un aspect avantageux de l'invention le rapport molaire entre l'équivalent unité N-vinylimidazole et l'actif hydrophile sensible à l'oxydation varie entre 0,004 et 16 et préférentiellement entre 0,01 et 1.

De façon préférentielle on utilisera un copolymère de N-vinylimidazole / N-vinylpyrrolidone.

La masse molaire moyenne en poids des polymères de N-vinylimidazole sera avantageusement comprise entre 1000 et $1 \times 10^7$ et de préférence entre 5000 et $5 \times 10^6$.

On pourra utiliser à cet effet, le copolymère vinylpyrrolidone/vinylimidazole (50/50) ayant une masse molaire moyenne en poids de 1 200 000 vendu sous la référence LUVITEC VPI 55K72W par la société BASF ou le copolymère vinylpyrrolidone/vinylimidazole (50/50) ayant une masse molaire moyenne en poids de 10 000 vendu sous la référence LUVITEC VPI 55K18P par la société BASF.

Le copolymère est présent dans la composition selon l'invention en quantité suffisante pour obtenir l'effet recherché, c'est à dire en quantité suffisante pour stabiliser l'actif hydrophile sensible à l'oxydation. De préférence le copolymère est présent à une concentration comprise entre 0,1 et 5 % en poids, par rapport au poids total de la phase aqueuse, et plus particulièrement à une concentration comprise entre 0,1 et 2 % en poids, par rapport au poids total de la phase aqueuse.

Les compositions utilisées selon l'invention sont destinées à une application topique sur la peau et/ou ses phanères et contiennent donc un milieu physiologiquement acceptable, c'est-à-dire compatible avec les tissus cutanés tels que la peau, le cuir chevelu, les cils, les sourcils, les cheveux, les ongles et les muqueuses. Ce milieu physiologiquement acceptable comprend une phase aqueuse et éventuellement d'un solvant organique physiologiquement acceptable choisi par exemple parmi les alcools inférieurs comportant de 1 à 8 atomes de carbone et en particulier de 1 à 6 atomes de carbone, comme l'éthanol, l'isopropanol, le propanol, le butanol ; les polyéthylène glycols ayant de 6 à 80 unités oxyde d'éthylène ; les polyols comme le propylène glycol, l'isoprène glycol, le butylène glycol, la glycérine, le sorbitol.

Quand le milieu physiologiquement acceptable est un milieu aqueux, il a généralement un pH compatible avec la peau, allant de préférence de 3 à 9 et mieux de 3,5 à 7,5.

Les compositions selon l'invention peuvent se présenter sous toutes les formes galéniques classiquement utilisées pour une application topique et notamment sous forme de solutions aqueuses, hydroalcooliques, d'émulsions huile-dans-eau (H/E) ou eau-dans-huile (E/H) ou multiple (triple : E/H/E ou H/E/H), de gels aqueux, ou de dispersions d'une phase grasse dans une phase aqueuse à l'aide de sphérules, ces sphérules pouvant être des nanoparticules polymériques telles que les nanosphères et les nanocapsules, ou des vésicules lipidiques de type ionique et/ou non ionique (liposomes, niosomes, oléosomes). Ces compositions sont préparées selon les méthodes usuelles.

En outre, les compositions utilisées selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick.

Quand la composition utilisée selon l'invention comporte une phase huileuse, celle-ci contient de préférence au moins une huile. Elle peut contenir en outre d'autres corps gras.

Comme huiles utilisables dans la composition de l'invention, on peut citer par exemple :

- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules $R^1COOR^2$ et $R^1OR^2$ dans laquelle $R^1$ représente le reste d'un acide gras comportant de 8 à 29 atomes de carbone, et $R^2$ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;

- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Selon un mode particulier de réalisation de l'invention, la composition selon l'invention est une émulsion eau-dans-huile (E/H) ou huile-dans-eau (H/E). La proportion de la phase huileuse de l'émulsion peut aller de 5 à 80 % en poids, et de préférence de 5 à 50 % en poids par rapport au poids total de la composition.

Les émulsions contiennent généralement au moins un émulsionnant choisi parmi les émulsionnants amphotères, anioniques, cationiques ou non ioniques, utilisés seuls ou en mélange, et éventuellement un co-émulsionnant. Les émulsionnants sont choisis de manière appropriée suivant l'émulsion à obtenir (E/H ou H/E). L'émulsionnant et le co-émulsionnant sont généralement présents dans la composition, en une proportion allant de 0,3 à 30 % en poids, et de préférence de 0,5 à 20 % en poids par rapport au poids total de la composition.

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination Abil EM 90R par la société Goldschmidt. On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu. On peut également utiliser comme émulsionnant un oligomère ou un polymère dérivé de polyoléfine à terminaison succinique, celui-ci est de préférence une polyoléfine à terminaison succinique estérifiée ou amidifiée, ou un sel d'une telle polyoléfine, et en particulier du polyisobutylène à terminaison succinique estérifiée ou amidifiée tels que les produits commercialisés sous les dénominations L5603 et L2721 et OS131769 par la société Lubrizol.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

De façon connue, la composition cosmétique ou dermatologique de l'invention peut contenir également des adjuvants habituels dans le domaine cosmétique ou dermatologique, tels que les gélifiants hydrophiles ou lipophiles, les conservateurs, les solvants, les parfums, les charges, les filtres UV, les bactéricides, les absorbeurs d'odeur, les matières colorantes, les extraits végétaux, les sels. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les sphérules lipidiques.

Comme charges qui peuvent être utilisées dans la composition de l'invention, on peut citer par exemple, outre les pigments, la poudre de silice ; le talc ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de la composition.

Selon un mode préféré de réalisation, les compositions conformes à l'invention peuvent comporter en plus au moins un agent photoprotecteur organique et/ou au moins un agent photoprotecteur inorganique actif dans l'UVA et/ou l'UVB (absorbeurs), hydrosolubles ou liposolubles ou bien insolubles dans les solvants cosmétiques couramment utilisés.

Les agents photoprotecteurs organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de $\beta,\beta$-diphénylacrylate; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US5,166,355, GB2303549, DE 197 26 184 et EP893119 ; les polymères filtres et silicones filtres tels que ceux décrits notamment dans la demande WO-93/04665 ; les dimères dérivés d'$\alpha$-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

A titre d'illustration, comme d'agents photoprotecteurs actifs dans l'UV-A et/ou l'UV-B, on peut citer, désignés ci-dessous sous leur nom INCI :

les dérivés de l'acide p-aminobenzoïque (PABA), en particulier le PABA, l'éthyl PABA, l'éthyl Dihydroxypropyl PABA, l'éthylhexyl Diméthyl PABA (vendu notamment sous le nom « ESCALOL 507 » par ISP), le glyceryl PABA, ou le PEG-25 PABA (vendu sous le nom « UVINUL P25 » par BASF),

les dérivés salicyliques, en particulier l'homosalate (vendu sous le nom « EUSOLEX HMS » par RONA/EM INDUSTRIES), l'éthylhexyl salicylate (vendu sous le nom « NEO HELIOPAN OS » par HAARMANN et REIMER), le dipropyleneglycol salicylate (vendu sous le nom « DIPSAL » par SCHER), ou le TEA salicylate (vendu sous le nom « NEO HELIOPAN TS » par HAARMANN et REIMER),

les dérivés de dibenzoylméthane, en particulier le butyl Methoxydibenzoylmethane (vendu notamment sous le nom commercial « PARSOL 1789 » par HOFFMANN LA ROCHE), ou l'isopropyl Dibenzoylmethane,

les dérivés cinnamiques, en particulier l'éthylhexyl Methoxycinnamate (vendu notamment sous le nom commercial « PARSOL MCX » par HOFFMANN LA ROCHE), l'isopropyl methoxy cinnamate, l'isoamyl Methoxy cinnamate (vendu sous le nom commercial « NEO HELIOPAN E 1000 » par HAARMANN et REIMER), le cinoxate, le DEA methoxycinnamate, le diisopropyl methyl cinnamate, ou le glyceryl ethylhexanoate dimethoxycinnamate,

les dérivés de $\beta,\beta$-diphénylacrylate, en particulier l'octocrylene (vendu notamment sous le nom commercial « UVINUL N539 » par BASF), ou l'étocrylene, (vendu notamment sous le nom commercial « UVINUL N35 » par BASF),

les dérivés de la benzophénone, en particulier la benzophenone-1 (vendue sous le nom commercial « UVINUL 400 » par BASF), la benzophenone-2 (vendue sous le nom commercial « UVINUL D50 » par BASF), la benzophenone-3 ou oxybenzone (vendu sous le nom commercial « UVINUL M40 » par BASF), la benzophenone-4 (vendue sous le nom commercial « UVINUL MS40 » par BASF), la benzophenone-5, la benzophenone-6 (vendue sous le nom commercial « HELISORB 11 » par NORQUAY), la benzophenone-8 (vendue sous le nom commercial « SPECTRA-SORB UV-24 » PAR AMERICAN CYANAMID), la benzophenone-9 (vendue sous le nom commercial « UVINUL DS-49» par BASF), la benzophenone-12, ou le 2-(4-diéthylamino-2-hydroxybenzoyl)-benzoate de n-hexyle,

les dérivés du benzylidène camphre, en particulier le 3-benzylidene camphor (fabriqué sous le nom « MEXORYL SD» par CHIMEX), le 4-methylbenzylidene camphor (vendu sous le nom « EUSOLEX 6300 » par MERCK), le benzylidene camphor Sulfonic Acid (fabriqué sous le nom « MEXORYL SL» par CHIMEX), le camphor benzalkonium methosulfate (fabriqué sous le nom « MEXORYL SO » par CHIMEX), le terephthalylidene dicamphor sulfonic acid (fabriqué sous le nom « MEXORYL SX » par CHIMEX), ou le polyacrylamidomethyl benzylidene camphor (fabriqué sous le nom « MESORYL SW » par CHIMEX),

les dérivés de benzimidazole, en particulier le phenylbenzimidazole sulfonic acid (vendu notamment sous le nom commercial « EUSOLEX 232 » par MERCK), ou le disodium phenyl dibenzimidazole tetra-sulfonate (vendu sous le nom commercial « NEO HELIOPAN AP » par HAARMANN et REIMER),

les dérivés de triazine, en particulier l'anisotriazine (vendue sous le nom commercial «TINOSORB S » par CIBA SPECIALTY CHEMICALS), l'éthylhexyl triazone (vendue notamment sous le nom commercial «UVINUL T150 » par BASF), la diethylhexyl butamido triazone (vendue sous le nom commercial « UVASORB HEB » par SIGMA 3V), ou la 2,4,6- tris-(4'amino-benzalmalonate de diisobutyle)-s-triazine,

les dérivés de benzotriazole, en particulier, le drometrizole trisiloxane (vendu sous le nom « SILATRIZOLE » par RHODIA CHIMIE), le methylène bis-benzotriazolyl tetramethylbutylphénol (vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS),

les dérivés anthraniliques, en particulier le menthyl anthranilate (vendu sous le nom commercial « NEO HELIOPAN MA » par HAARMANN et REIMER),

les dérivés d'imidazolines, en particulier l'éthylhexyl dimethoxybenzylidene dioxoimidazoline propionate,

les dérivés de benzalmalonate, en particulier le polyorganosiloxane à fonctions benzalmalonate (vendu sous la dénomination commerciale « PARSOL SLX » par HOFFMANN LA ROCHE),

les dérivés de 4,4-diarylbutadiène, en particulier le 1,1'-dicarboxy (2,2'-diméthylpropyl)-4,4-diphénylbutadiène,

et leurs mélanges.

Les agents photoprotecteurs organiques plus particulièrement préférés sont choisis parmi l'éthylhexyl salicylate, l'éthylhexyl methoxycinnamate, l'octocrylene, le phenylbenzimidazole sulfonic acid, la benzophenone-3, la benzophenone-4, la benzophenone-5, la 4-methylbenzylidene camphor, le terephthalylidene dicamphor sulfonic acid, le disodium phenyl dibenzimidazole tetra-sulfonate, la 2,4,6-tris-(4'-amino benzalmalonate de diisobutyle)-s-triazine, l'anisotriazine, l'éthylhexyl triazone, la diethylhexyl butamido triazone, le methylène bis-benzotriazolyl tetramethylbutylphénol, le drometrizole trisiloxane, le 1,1'-dicarboxy (2,2'-diméthyl-propyl)-4,4-diphénylbutadiène, et leurs mélanges.

Les agents photoprotecteurs inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les agents photoprotecteurs sont généralement présents dans les compositions selon l'invention dans des proportions allant de 0,1 à 20% en poids par rapport au poids total de la composition, et de préférence allant de 0,2 à 15% en poids par rapport au poids total de la composition.

Dans un autre aspect avantageux de l'invention, la composition utilisée peut contenir en outre au moins un autre agent choisi parmi les agents piégeurs d'ozone, les agents piégeurs de métaux lourds, et les agents anti-radicalaires.

Comme agents piégeurs d'ozone utilisables dans la composition selon l'invention, on peut citer en particulier les phénols et polyphénols, en particulier les tannins, l'acide ellagique et l'acide tannique ; l'épigallocatéchine et les extraits naturels en contenant ; les extraits de feuille d'olivier ; les extraits de thé, en particulier de thé vert ; les anthocyanes ; les extraits de romarin ; les acides phénols, en particulier l'acide chorogénique ; les stilbènes, en particulier le resvératrol ; les dérivés d'acides aminés soufrés, en particulier la S-carboxyméthylcystéine ; l'ergothionéine ; la N-acétylcystéine ; des chélatants comme la N,N'-bis-(3,4,5-triméthoxybenzyl)éthylènediamine ou l'un de ses sels, complexes métalliques ou esters ; des caroténoïdes tels que la crocétine ; et des matières premières diverses comme le mélange d'arginine, ribonucléate d'histidine, mannitol, adénosinetriphosphate, pyridoxine, phénylalanine, tyrosine et ARN hydrolysé commercialisé par les Laboratoires Sérobiologiques sous la dénomination commerciale CPP LS 2633-12F®, la fraction hydrosoluble de maïs commercialisée par la société SOLABIA sous la dénomination commerciale Phytovityl®, le mélange d'extrait de fumeterre et d'extrait de citron commercialisé sous la dénomination Unicotrozon C-49® par la société Induchem, et le mélange d'extraits de ginseng, de pomme, de pêche, de blé et d'orge vendu par la société PROVITAL sous la dénomination commerciale Pronalen Bioprotect®.

Enfin, comme agents piégeurs de métaux lourds utilisables dans la composition selon l'invention, on peut citer en particulier les agents chélatants tels que l'EDTA, le sel pentasodique de l'acide éthylènediamine tétraméthylène phosphonique, et la N,N'-bis-(3,4,5-triméthoxybenzyl)éthylènediamine ou l'un de ses sels, complexes métalliques ou esters ; l'acide phytique ; les dérivés de chitosan ; les extraits de thé, en particulier de thé vert ; les tannins tels que l'acide ellagique ; les acides aminés soufrés tels que la cystéine ; les extraits de Jacinthe d'eau (Eichornia crassipes) ; et la fraction hydrosoluble de maïs commercialisée par la société SOLABIA sous la dénomination commerciale Phytovityl®.

Les agents anti-radicalaires utilisables dans la composition selon l'invention comprennent, outre certains agents anti-pollution mentionnés précédemment, la vitamine E et ses dérivés tels que l'acétate de tocophéryle ; les bioflavonoïdes ; le co-enzyme Q10 ou ubiquinone ; certaines enzymes comme la catalase, le superoxyde dismutase, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; les lignanes ; et la mélatonine.

La composition selon l'invention peut être appliquée sur la peau ou les lèvres. Elle peut ainsi être utilisée dans un procédé de traitement cosmétique en vue de prévenir ou lutter contre les effets néfastes des UV et/ou de la pollution sur la peau ou les muqueuses, comprenant l'application de la composition selon l'invention sur la peau ou les muqueuses.

L'invention concerne également un procédé de traitement cosmétique en vue de prévenir ou lutter contre la perte de fermeté et/ou d'élasticité de la peau ou des muqueuses, comprenant l'application de la composition selon l'invention sur la peau ou les muqueuses.

En variante, la composition selon l'invention peut être utilisée pour la fabrication d'une préparation dermatologique comprenant une phase aqueuse, destinée à prévenir ou lutter contre les effets néfastes des UV et/ou de la pollution sur la peau.

Dans une autre variante, la composition selon l'invention peut être utilisée pour la fabrication d'une préparation dermatologique comprenant une phase aqueuse, destinée à prévenir ou lutter contre la perte de fermeté et/ou d'élasticité de la peau.

Les exemples qui suivent servent à illustrer l'invention sans toutefois présenter un caractère limitatif. Les composés sont, selon le cas, cités en noms chimiques ou en noms CTFA (International Cosmetic Ingredient Dictionary and Handbook).

## EXEMPLES

**Exemple 1 : Test de conservation accéléré.**

Ce test a pour but d'étudier la dégradation d'un actif hydrophile sensible à l'oxydation après deux mois de conservation à 45°C. Diverses solutions ont été réalisées, et leurs compositions sont regroupées dans le tableau suivant :

Tableau I :

| Compositions (dans l'eau) | solution A (Témoin) | solution B | solution C | solution D |
|---|---|---|---|---|
| acide ascorbique | 15% | 15 % | 15% | 15% |
| polymère 1 | - | 1 % | - | - |

Tableau I : (suite)

| Compositions (dans l'eau) | solution A (Témoin) | solution B | solution C | solution D |
|---|---|---|---|---|
| polymère 2 | - | - | 1% | - |
| polymère 3 | - | - | - | 1% |

Toutes les solutions sont ramenées à pH 6 avec KOH 8,9 mole/l

Les pourcentages des polymères sont donnés en matière active.

Polymère 1 : Copolymère vinylpyrrolidone-vinylimidazole (50/50) vendu sous la référence LUVITEC VPI 55K72W de la société BASF (Masse mol. moyenne en poids-1,2.10$^6$).

Polymère 2 : Copolymère vinylpyrrolidone-vinylimidazole (50/50) vendu sous la référence LUVITEC VPI 55K18P de la société BASF (Masse mol. moyenne en poids 10000).

Polymère 3 : Polyvinylpyrrolidone vendu sous la référence KOLLIDON 12PF de la société BASF (Masse mol. moyenne en poids 3000).

Le taux de dégradation mesuré est donné par le rapport

$$(C_0 - C_{2mois})/C_0$$

avec $C_0$ concentration en acide ascorbique à t=0 et $C_{2mois}$ la concentration en acide ascorbique à t=2 mois, dans les conditions indiquées dans le tableau ci-dessus.

La concentration en acide ascorbique est déterminée par la technique HPLC (système LaChrom Merck). Les conditions analytiques sont les suivantes :

Colonne Lichrosphere100 RP18 (250 mm)
Eluant : tampon phosphate O,1M, pH 2,1
Débit : 1 ml/min.
Détection à 257 nm

Dilution de l'échantillon tel que la concentrattion en acide ascorbique soit comprise entre 0,05 et 1 mg/ml.

Les résultats obtenus sont regroupés dans le tableau II suivant :

Tableau II :

|  | Taux de dégradation après 2 mois à 45°C (en %) | |
|---|---|---|
|  | sous air, flacon verre ambré | sous azote, flacon aluminium |
| solution A | 43 | 19,4 |
| solution B | 10,8 | 1 |
| solution C | 23,4 | 4,5 |
| solution D | 35,8 | 15,7 |

On constate d'après le tableau II que la stabilité de l'acide ascorbique est améliorée en présence des polymères 1 et 2 de l'invention, même en présence de l'oxygène de l'air, comparativement au témoin. On constate également que l'homopolymère de N-vinylpyrrolidone seul ne suffit pas à stabiliser de façon efficace la solution d'acide ascorbique.

Les polymères cités étant hydrophiles, il suffira de les ajouter à une solution aqueuse d'acide ascorbique pour stabiliser ce dernier.

**Exemple 2 : mise en évidence de l'acitivité anti-radicalaire :**

**I. Principe**

Ce test permet d'évaluer l'effet anti-OH° d'une molécule.

Il est basé sur la mesure en chromatographie en phase gazeuse de l'éthylène formé à partir de l'oxydation de la méthionine par le radical hydroxyle. Celui-ci est généré par une réaction de Fenton ferro catalysée, entretenue par la génération continue d'anions superoxydes. Les anions $O2^{°-}$ sont produits par réduction photochimique à 365 nm de la riboflavine (RBF) par un donneur d'hydrogène, suivant le schéma suivant :

$$RBF \xrightarrow{\text{UV 365 nm}} {}^3RBF \xrightarrow{\text{NADH}} RBFH_2$$

$$RBFH_2 + O_2 \rightarrow RBF + 2\,O_2^{°-} + 2\,H^+$$

$$2\,O_2^{°-} + 2H^+ \rightarrow H_2O_2 + O_2$$

$$O_2^{°-} + L\text{-}Fe^{3+} \rightarrow O_2 + L\text{-}Fe^{2+}$$

$$H_2O_2 + L\text{-}Fe^{2+} \rightarrow L\text{-}Fe^{3+} + OH\text{-} + OH^{o}$$

$$CH_3\text{-}S\text{-}CH_2\text{-}CH\,(COOH)\text{-}NH_2 + OH^{o} \rightarrow CH_2 = CH_2 + \text{produits}$$

La neutralisation des radicaux OH° se traduit par une inhibition de la production d'éthylène.

**II. Mode opératoire**

Matériel d'irradiation : 3 tubes à vapeur de mercure basse pression.

Les produits testés sont solubilisés dans un tampon phosphate. Ils sont testés à des concentrations finales dans le mélange réactionnel allant généralement de 0,1 à 3 %, suivant leur solubilité, le volume final est de 2 ml.

On règle la hauteur d'exposition sous la rampe UV afin d'avoir un temps d'exposition d'environ 8 minutes pour une dose d'UVA de 1 joule / cm$^2$ et de ne pas modifier ce réglage pendant toute la durée du test.

Dans un flacon head-space, on introduit dans l'ordre suivant :

◊    1,4 ml de tampon phosphate
◊    100 μl de solution de méthionine 200mM
◊    100 μl de solution de chlorure ferrique 4mM
◊    100 μl de tampon phosphate pH 7,4 (témoin) ou de solution contenant l'actif à tester
◊    100 μl de solution d'EDTA 4mM
◊    100 μl de solution de NADH 400mM

Les échantillons et les placebo sont tous préparés à la suite et gardés à l'abri de la lumière.

La rampe UVA est allumée en affichant un nombre de joules au moins égal au nombre d'échantillons, les échantillons sont irradiés un par un à 0,5 Joules d'intervalle.

Tous les 0,5 joules :

◊   Ajouter 100 µl de riboflavine
◊   mélanger
◊   Irradier 1 joule
◊   Arrêter avec 0,5 ml de NaOH 1N
◊   Mettre à l'abri de la lumière

Les flacons sont insérés dans le passeur d'échantillons du chromatographe. Le pic d'éthylène sort à un temps de rétention proche de 2,00 minutes. Effectuer au minimum 3 mesures par échantillon.

### III. Résultats :

Les résultats sont exprimés en pourcentage d'inhibition de production d'éthylène par rapport à la solution témoin.
Il apparaît que, pour les échantillons testés contenant l'association acide ascorbique et copolymère N-vinylimidazole / N-vinylpyrrolidone, l'inhibition de la production d'éthylène est augmentée par rapport au témoin, mettant ainsi en évidence l'activité antiradicalaire de cette association.

**Exemple 3 :** crème H/E

On prépare la composition suivante de manière classique pour l'homme du métier.

| | |
|---|---|
| Glyceryl stearate and Peg-100 stearate | 2,5 g |
| Peg-50 stearate | 2,5 g |
| Cetyl alcohol | 1 g |
| Stearyl alcohol | 1 g |
| Hydrogenated polyisobutene | 5 g |

| | |
|---|---|
| Eau | 12,23 g |
| Glycerin | 5 g |

| | |
|---|---|
| Cyclopentasiloxane | 15 g |
| Carbomer | 0,6 g |
| Phenoxyethanol | 1 g |

| | |
|---|---|
| Eau | 45,17 g |
| Ascorbic acid | 5 g |
| Potassium hydroxide (50% solution) | 3 g |
| Vinylpyrrolidone/vinylimidazole copolymer | 1 g |

Cette crème douce à l'application permet de lisser les traits du visage et présente un bonne stabilité pour l'acide ascorbique.

**Exemple 4 :** émulsion E/H

On prépare la composition suivante de manière classique pour l'homme du métier.

| | |
|---|---|
| Eau | 45,17 g |
| Ascorbic acid | 5 g |
| Potassium hydroxide (50% solution) | 3 g |
| Vinylpyrrolidone/vinylimidazole copolymer | 1 g |

(suite)

| Glycerin | 5 g |
|---|---|
| Phenoxyethanol | 1 g |

| Cyclopentasiloxane and dimethicone copolyol | 20 g |
|---|---|
| Phenyl trimethicone | 4 g |
| Prunus armeniaca (apricot) kernel oil | 3,5 g |
| Dimethicone and Dimethicone/vinyl dimethicone crosspolymer | 5 g |
| Nylon-12 | 5 g |

On obtient une émulsion eau-dans-huile blanche, apte à lisser les traits du visage, et dans laquelle l'acide ascorbique présente une bonne stabilité.

**Revendications**

1. Utilisation cosmétique pour prévenir ou lutter contre les effets néfastes des UV et/ou de la pollution sur la peau, d'une composition contenant, dans un milieu physiologiquement acceptable comprenant une phasse aqueuse, au moins un actif hydrophile sensible à l'oxydation choisi parmi l'acide ascorbique et ses dérivés et au moins un polymère ou copolymère de N-vinylimidazole non réticulé, ledit actif et ledit polymère ou copolymère étant tous deux dans la phase aqueuse.

2. Utilisation cosmétique pour prévenir ou lutter contre la perte de fermeté et/ou d'élasticité de la peau, d'une composition contenant, dans un milieu physiologiquement acceptable comprenant un phase aqueuse, au moins un actif hydrophile sensible à l'oxydation choisi parmi l'acide ascorbique et ses dérivés et au moins un polymère ou copolymère de N-vinylimidazole non réticulé, ledit actif et ledit polymère étant tous deux dans la phase aqueuse.

3. Utilisation d'une association constituée d'au moins un actif hydrophile sensible à l'oxydation choisi parmi l'acide ascorbique et ses dérivés et d'au moins un polymère ou copolymère de N-vinylimidazole non réticulé, dans la phase aqueuse d'une composition cosmétique, en tant qu'agent antiradicalaire.

4. Utilisation d'une association constituée d'au moins un actif hydrophile sensible à l'oxydation choisi parmi l'acide ascorbique et ses dérivés et d'au moins un polymère ou copolymère de N-vinylimidazole non réticulé, dans la phase aqueuse d'une composition cosmétique, en tant qu'agent apaisant des érythèmes solaires.

5. Utilisation d'au moins un actif hydrophile sensible à l'oxydation choisi parmi l'acide ascorbique et ses dérivés et d'au moins un polymère ou copolymère de N-vinylimidazole non réticulé pour la préparation d'une composition dermatologique comprenant une phase aqueuse, destinée à prévenir ou lutter contre les effets néfastes des UV et/ou de la pollution sur la peau.

6. Utilisation selon l'une quelconque des revendications 1 à 5, **caractérisée en ce que** l'actif hydrophile est choisi parmi les dérivés de l'acide ascorbique tels que le 5,6-di-O-diméthylsilyl-ascorbate, le dl-alpha-tocopheryl-dl-ascorbyl-phosphate sel de potassium, l'ascorbyl phosphate de magnésium, et l'ascorbyl phosphate de sodium.

7. Utilisation selon l'une quelconque des revendications 1 à 5, **caractérisée en ce que** l'actif hydrophile sensible à l'oxydation est l'acide ascorbique.

8. Utilisation selon l'une quelconque des revendications 1 à 7, **caractérisée en ce que** le copolymère non réticulé est une association de sous unités N-vinylimidazole avec des sous unités N-vinylpyrrolidone et/ou N-vinylcaprolactam.

9. Utilisation selon la revendication 8, **caractérisée en ce que** le copolymère non réticulé est un copolymère de N-vinylimidazole / N-vinylpyrrolidone.

**10.** Utilisation selon l'une quelconque des revendications 1 à 9, **caractérisée en ce que** le copolymère non réticulé est choisi parmi un copolymère vinylpyrrolidone/vinylimidazole (50/50) ayant une masse molaire moyenne en poids de 1 200 000 et un copolymère vinylpyrrolidone/vinylimidazole (50/50) ayant une masse molaire moyenne en poids de 10 000.

**11.** Utilisation selon l'une quelconque des revendications 1 à 10, **caractérisée en ce que** le rapport molaire entre l'équivalent unité N-vinylimidazole et l'actif hydrophile sensible à l'oxydation varie entre 0,004 et 16.

**12.** Utilisation selon la revendication 11, **caractérisée en ce que** le rapport molaire entre l'équivalent unité N-vinylimidazole et l'actif hydrophile sensible à l'oxydation varie entre 0,01 et 1.

**13.** Utilisation selon l'une quelconque des revendications 1 à 12, **caractérisée en ce que** le polymère ou copolymère est présent à une concentration comprise entre 0,1 et 5 % en poids de la phase aqueuse.

**14.** Utilisation selon la revendication 13, **caractérisée en ce que** le polymère ou copolymère est présent à une concentration comprise entre 0,1 et 2 % en poids de la phase aqueuse.

**15.** Utilisation selon l'une quelconque des revendications 1 à 14 **caractérisée en ce que** le polymère ou copolymère possède une fraction molaire en unité N-vinylimidazole comprise entre 0,1 et 1.

**16.** Utilisation selon la revendication 15, **caractérisée en ce que** le polymère ou copolymère possède une fraction molaire en unité N-vinylimidazole comprise entre 0,4 et 0,9.

**17.** Utilisation selon l'une quelconque des revendications 1 à 16, **caractérisée en ce que** la composition contient en outre un autre agent choisi parmi les agents piégeurs d'ozone, les agents piégeurs de métaux lourds, et les agents anti-radicalaires.

**18.** Procédé de traitement cosmétique destiné à prévenir ou lutter contre les effets néfastes des UV et/ou de la pollution sur la peau ou les muqueuses, comprenant l'application sur la peau ou les muqueuses d'une composition contenant, dans un milieu physiologiquement acceptable comprenant une phase aqueuse, au moins un actif hydrophile sensible à l'oxydation choisi parmi l'acide ascorbique et ses dérivés et au moins un polymère ou copolymère de N-vinylimidazole non réticulé, ledit actif et ledit polymère étant tous deux dans la phase aqueuse.

**19.** Procédé cosmétique destiné à lutter contre la perte de fermeté et/ou d'élasticité de la peau ou des muqueuses, comprenant l'application sur la peau ou les muqueuses d'une composition contenant, dans un milieu physiologiquement acceptable comprenant une phase aqueuse, au moins un actif hydrophile sensible à l'oxydation choisi parmi l'acide ascorbique et ses dérivés et au moins un polymère ou copolymère de N-vinylimidazole non réticulé, ledit actif et ledit polymère étant tous deux dans la phase aqueuse.

| | Office européen des brevets | RAPPORT DE RECHERCHE EUROPEENNE | Numéro de la demande EP 02 29 2809 |

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | EP 1 064 924 A (BASF AG) 3 janvier 2001 (2001-01-03) * revendication 1; exemple 10 * --- | 1 | A61K7/48 |
| A | US 5 922 758 A (D. BISSETT) 13 juillet 1999 (1999-07-13) * colonne 8, ligne 24-50; revendication 1 * ----- | 1 | |

**DOMAINES TECHNIQUES RECHERCHES (Int.Cl.7)**

A61K
A61Q

Le présent rapport a été établi pour toutes les revendications

| Lieu de la recherche | Date d'achèvement de la recherche | Examinateur |
|---|---|---|
| LA HAYE | 31 janvier 2003 | Glikman, J-F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 02 29 2809

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

31-01-2003

| Document brevet cité au rapport de recherche | | Date de publication | Membre(s) de la famille de brevet(s) | | Date de publication |
|---|---|---|---|---|---|
| EP 1064924 | A | 03-01-2001 | DE | 19929758 A1 | 04-01-2001 |
| | | | BR | 0002906 A | 30-01-2001 |
| | | | CN | 1282571 A | 07-02-2001 |
| | | | EP | 1064924 A2 | 03-01-2001 |
| | | | JP | 2001055321 A | 27-02-2001 |
| US 5922758 | A | 13-07-1999 | US | 5739156 A | 14-04-1998 |

Pour tout renseignement concernant cette annexe : voir Journal Officiel de l'Office européen des brevets, No.12/82